# EUROPÄISCHE PATENTSCHRIFT

(11) **EP 0 731 075 B1**
(45) Veröffentlichungstag und Bekanntmachung des Hinweises auf die Patenterteilung: **08.12.1999**
(21) Anmeldenummer: 96102652.3
(22) Anmeldetag: 22.02.1996
(51) Int. Cl.: C07C 45/00, C07C 231/14

(54) **Verfahren zur Herstellung von substituierten Cyclohexanonen**
Process for the preparation of substituted cyclohexanones
Procédé pour la préparation de cyclohexanones substituées

(30) Priorität: 06.03.1995 DE 19507752
(43) Veröffentlichungstag der Anmeldung: 11.09.1996
(73) Patentinhaber: BAYER AG, 51368 Leverkusen (DE)
(72) Erfinder: Landscheidt, Heinz, Dr., 47057 Duisburg (DE); Klausener, Alexander, Dr., 50670 Köln (DE); Zirngiebel, Eberhard, Dr., 51061 Köln (DE); Kiel, Wolfgang, Dr., 51519 Odenthal (DE)

(56) Entgegenhaltungen:
- CH-A- 189 403
- US-A- 3 124 614
- US-A- 3 193 584
- US-A- 4 503 273

## Beschreibung

Die vorliegende Erfindung betrifft ein Verfahren zur Herstellung substituierter Cyclohexanone durch katalytische Hydrierung der zugrundeliegenden substituierten Phenole in einem Lösungsmittel aus der Gruppe der Ether.

Substituierte Cyclohexanone sind als Zwischenprodukte für die Herstellung von Farbstoffen, pharmazeutischen Wirkstoffen und Pflanzenschutzmitteln von Bedeutung. So läßt sich, ausgehend von 4-Hydroxy-cyclohexanon, nach US 3.965.180 in wenigen Schritten eine pharmazeutisch wirksame Verbindung synthetisieren; in EP 186.087 wird die Herstellung einer Verbindung mit pharmakologischen Eigenschaften, ausgehend von 4-Acetylamino-cyclohexanon, beschrieben.

Die Herstellung der genannten substituierten Cyclohexanone nach dem Stand der Technik wird im allgemeinen im Zuge mehrerer aufeinanderfolgender Syntheseschritte durchgeführt. Solche Verfahren bedingen einen hohen Aufwand an dazu erforderlichen Chemikalien und Apparaturen und sind daher in aller Regel unter ökologischen und ökonomischen Gesichtspunkten ungünstig zu beurteilen. So wird in Synth. Comm. 4 (1974), S. 155-159, die Herstellung von 4-Hydroxy-cyclohexanon durch Oxidation von 1,4-Cyclohexandiol mit Chromsäure beschrieben, wobei eine Raumausbeute von nur 10 g/l erzielt wird. Diese geringe Raum-Zeit-Ausbeute, verbunden mit der Schwermetallproblematik, läßt eine technische Umsetzung dieses Verfahrens nicht zu.

Es ist seit langem bekannt, daß sich das unsubstituierte Cyclohexanon durch heterogen katalysierte Hydrierung von Phenol in der Schmelze herstellen läßt (US 2.829.166; DE 2.752.291). Die Übertragung dieser Verfahrensweise auf substituierte Phenole ist jedoch nur in wenigen Fällen und in häufig nicht zufriedenstellenden Selektivitäten und Ausbeuten gelungen. So wird in DE 2.909.780 am Beispiel der Herstellung von 4-tert.-Amyl-cyclohexanon aus 4-tert.-Amylphenol die heterogen katalysierte Hydrierung von 4-Alkylphenolen zu 4-Alkyl-cyclohexanonen in der Schmelze beschrieben. Da zur erfolgreichen Durchführung dieser Umsetzung die Reaktionstemperatur in einem für die selektive Hydrierung günstigen Temperaturbereich von 140 bis 200°C liegen muß, kommen für ein derartiges Verfahren nur substituierte Phenole mit einem entsprechend niedrigen Schmelzpunkt in Frage. Weitere Einschränkungen ergeben sich dadurch, daß substituierte Phenole, die empfindliche Gruppen tragen, unter den für das Arbeiten in der Schmelze notwendigen Temperaturen Nebenreaktionen eingehen können.

In JP 82.004.932 (zitiert nach C.A. 96: 199.167v) wird die heterogen katalysierte Hydrierung substituierter Phenole in wäßriger Lösung beschrieben. Dabei werden aber nur äußerst geringe und unter ökonomischen Gesichtspunkten uninteressante Raum-Zeit-Ausbeuten erzielt. So werden in den Patentbeispielen 50 mmol substituiertes Phenol in 200 ml Wasser umgesetzt. Ein weiterer schwerwiegender Nachteil einer derartigen Verfahrensweise ergibt sich daraus, daß das gewünschte Reaktionsprodukt nur durch aufwendige Extraktion mit einem organischen Lösungsmittel aus dem Reaktionsansatz abgetrennt werden kann, was im Falle einer technischen Realisierung zu einem hohen zusätzlichen Aufwand führt.

Neben den oben beispielhaft zitierten heterogen katalysierten Hydrierungen in der Schmelze bzw. in wäßriger Lösung wurden auch andere Lösungsmittel eingesetzt. Dieses Vorgehen ist jedoch, folgt man den Angaben der Literatur (JP 82/004, 932), ebenfalls mit Nachteilen verbunden, da im allgemeinen bei der heterogen katalysierten Hydrierung substituierter Phenole nur eine schlechte Selektivität bezüglich des gewünschten substituierten Cyclohexanons erzielt wird. So wird in den Vergleichsbeispielen der obengenannten Patentanmeldung gezeigt, daß gängige organische Lösungsmittel, wie Ethanol oder Essigsäure, völlig ungeeignet sind.

Ähnliche Beobachtungen wurden auch in verschiedenen wissenschaftlichen Artikeln beschrieben. So wird in Zh. Prikl. Khim. 52 (1979) 1823-6 (zitiert nach C.A. 92: 58.278n) ausgeführt, daß bei der Hydrierung von 4-tert.-Butylphenol die Ausbeute an Keton sinkt, wenn die Reaktion in einem Lösungsmittel durchgeführt wird. Wie die Autoren annehmen, verhindert die Anwesenheit des Lösungsmittels die Assoziation des Phenols mit dem Keton, wodurch dieses weiter zum Alkohol hydiert werden kann.

Es bestand daher weiterhin die Aufgabe, ein Verfahren zu finden, das die Hydrierung von substituierten Phenolen zu substituierten Cyclohexanonen in hohen Ausbeuten, Selektivitäten und Raum-Zeit-Ausbeuten ermöglicht. Die Erfindung betrifft ein Verfahren zur Herstellung von substituierten Cyclohexanonen der Formel 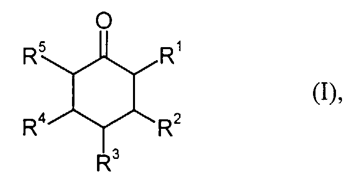 in der
- R¹, R², R⁴ und R⁵: für Wasserstoff und R³ für Hydroxy, C₁-C₄-Alkoxy oder -NH-C₁-C₄-Acyl stehen,
durch heterogen katalysierte Hydrierung von substituierten Phenolen der Formel 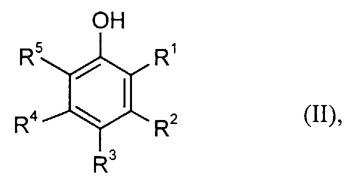 in der
- R¹, R², R³, R⁴ und R⁵: die oben angegebene Bedeutung haben,
das dadurch gekennzeichnet ist, daß die Hydrierung in Gegenwart eines gegebenenfalls auf einem Träger aufgebrachten Palladium-Katalysators sowie in Gegenwart eines oder mehrerer weiterer Zusatzstoffe aus der Reihe der alkalisch reagierenden (Erd)Alkali- oder Ammoniumsalze in einem Ether als Lösungsmittel durchgeführt wird.

C₁-C₄-Alkoxy ist beispielsweise Methoxy, Ethoxy, Propoxy, Isopropoxy, Butoxy, Isobutoxy, sec.-Butoxy oder tert.-Butoxy, bevorzugt Methoxy oder Ethoxy, besonders bevorzugt Methoxy.

C₁-C₄-Acyl ist beispielsweise Formyl, Acetyl, Propionyl, n-Butyryl oder i-Butyryl, bevorzugt Acetyl.

In besonders bevorzugter Weise wird ein Phenol der Formel 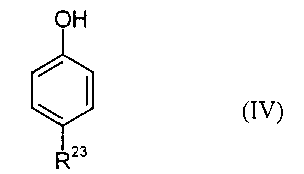 eingesetzt in der
- R²³: Hydroxy, Methoxy, Ethoxy oder Acetamido bedeutet.

Zur Durchführung des erfindungsgemäßen Verfahrens wird das substituierte Phenol (II) in einem Ether als Lösungsmittel, insbesondere in Diethylenglykoldimethylether, Ethylenglykoldimethylether, Dioxan oder Tetrahydrofuran in einem Gewichtsverhältnis von 2:1 bis 1:10 (Phenol/Lösungsmittel), gegebenenfalls bei erhöhter Temperatur, gelöst bzw. mit diesem gemischt und mit einem gegebenenfalls auf einem festen Trägermaterial, wie Aktivkohle, Al₂O₃, SiO₂ u.a., aufgebrachten Palladium-Katalysator in einem Gewichtsverhältnis von 10.000:1 bis 10:1(Phenol/Katalysator), sowie mit einem Zusatzstoff in einem Verhältnis von 20.000:1 bis 20:1 (Phenol/Aktivierungsmittel) versetzt.
In bevorzugter Weise wird Palladium auf Aktivkohle eingesetzt.
Als Zusatzstoffe einsetzbare alkalisch reagierende Salze sind beispielsweise die Hydroxide, Hydride, Carbonate, Hydrogencarbonate, Sulfite, Sulfide, Phosphate, Hydrogenphosphate, Boranate, Borate, C₁-C₆-Carboxylate von Li, Na, K, Rb, Cs, Mg, Ca, Sr, Ba, NH₄⁺ oder substituiertem NH₄⁺, bevorzugt die Carbonate, Hydrogencarbonate, Borate, Formiate und Acetate von Na, K, Ca, Mg, z.B. Natriumcarbonat und Borax.

Die erfindungsgemäße Hydrierung erfolgt unter Rühren bei einer Temperatur von 20°C bis 250°C, bevorzugt 60°C bis 230°C, besonders bevorzugt 100°C bis 210°C und

In besonders bevorzugter Weise wird ein Phenol der Formel 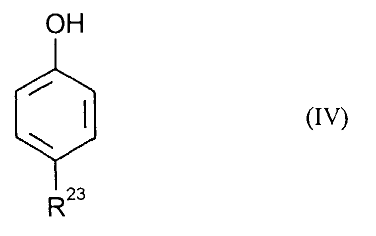 eingesetzt, in der
- R²³: Hydroxy, Methoxy, Ethoxy, Methylamino, Dimethylamino oder Acetamido bedeutet.

Zur Durchführung des erfindungsgemäßen Verfahrens wird das substituierte Phenol (II) in einem Ether als Lösungsmittel, insbesondere in Diethylenglykoldimethylether, Ethylenglykoldimethylether, Dioxan oder Tetrahydrofuran in einem Gewichtsverhältnis von 2:1 bis 1:10 (Phenol/Lösungsmittel), gegebenenfalls bei erhöhter Temperatur, gelöst bzw. mit diesem gemischt und mit einem gegebenenfalls auf einem festen Trägermaterial, wie Aktivkohle, Al₂O₃, SiO₂ u.a., aufgebrachten Katalysator aus der Gruppe der Metalle der Gruppe VIIIB des Periodensystems der Elemente (Mendelejew), in einem Gewichtsverhältnis von 10.000:1 bis 10:1 (Phenol/Katalysator), sowie gegebenenfalls mit einem Zusatzstoff in einem Verhältnis von 20.000:1 bis 20:1 (Phenol/Aktivierungsmittel) versetzt.

Metalle der Gruppe VIIIB des Periodensystems sind beispielsweise Palladium, Ruthenium, Rhodium, Platin, Nickel, bevorzugt Palladium. In bevorzugter Weise wird Palladium auf einem Träger, besonders bevorzugt auf Aktivkohle, eingesetzt.

Als Zusatzstoffe einsetzbare alkalisch reagierende Salze sind beispielsweise die Hydroxide, Hydride, Carbonate, Hydrogencarbonate, Sulfite, Sulfide, Phosphate, Hydrogenphosphate, Boranate, Borate, C₁-C₆-Carboxylate von Li, Na, K, Rb, Cs, Mg, Ca, Sr, Ba, NH₄⁺ oder substituiertem NH₄⁺, bevorzugt die Carbonate, Hydrogencarbonate, Borate, Formiate und Acetate von Na, K, Ca, Mg, z.B. Natriumcarbonat und Borax.

Die erfindungsgemäße Hydrierung erfolgt unter Rühren bei einer Temperatur von 20°C bis 250°C, bevorzugt 60 bis 230°C, besonders bevorzugt 100 bis 210°C und einem Wasserstoffdruck von 1 bar bis 200 bar, bevorzugt 2 bis 150 bar, besonders bevorzugt 3 bis 100 bar. Bei der Hydrierung wird zur Erzielung der optimalen Selektivität die aufgenommene Wasserstoffmenge zweckmäßigerweise aufgezeichnet, um bei Erreichen der vorberechneten Wasserstoffmenge von 1,5 bis 2,5 Mol Wasserstoff je Mol Phenol die Hydrierung abbrechen zu können. Dies kann durch Erniedrigung der Rührerdrehzahl, Absenken der Temperatur und/oder Unterbrechung der Wasserstoffzufuhr erreicht werden.

Nach Beendigung der erfindungsgemäßen Hydrierung wird der Katalysator mit Hilfe üblicher Techniken, beispielsweise durch Filtration, abgetrennt.

Das Lösungsmittel kann ebenfalls mit Hilfe üblicher Techniken, beispielsweise durch Destillation, abgetrennt und gegebenenfalls zurückgeführt werden, das Produkt kann auf an sich bekannte Weise, etwa durch Destillation oder Kristallisation, gereinigt werden.

Grundsätzlich ist es auch möglich und kann gegebenenfalls von Vorteil sein, die nach Beendigung der erfindungsgemäßen Hydrierung und Abtrennung des Katalysators erhaltene Lösung direkt, d.h. ohne weitere Aufarbeitung, für nachfolgende Umsetzungen wie beispielsweise Acetalisierung, Oximierung etc. einzusetzen.

### Beispiele

### Beispiel 1

Ein Gemisch aus 150 g Hydrochinon und 150 ml Diethylenglykoldimethylether wurde in Gegenwart von 3 g Pd (5 Gew.-%) auf Aktivkohle und unter Zusatz von 0,5 g Borax bei 160°C und einem Druck von 10 bar hydriert. Nach Aufnahme von 90 1 Wasserstoff wurde die Hydrierung unterbrochen. Der gaschromatographischen Analyse zufolge war 4-Hydroxy-cyclohexanon in einer Selektivität von 80 % entstanden. Durch Destillation ließ sich reines 4-Hydroxy-cyclohexanon in einer Menge von 65 % der theoretischen Ausbeute erhalten.

### Beispiel 2

Ein Gemisch aus 150 g 4-Methoxyphenol und 150 ml Diethylenglykoldimethylether wurde in Gegenwart von 3 g Pd (5 Gew.-%) auf Aktivkohle und unter Zusatz von 0,5 g Borax bei 160°C und einem Druck von 10 bar hydriert. Nach Aufnahme von 90 l Wasserstoff wurde die Hydrierung unterbrochen. Der gaschromatographischen Analyse zufolge war 4-Methoxycyclohexanon in einer Selektivität von 80 % entstanden. Durch Destillation ließ sich reines 4-Methoxycyclohexanon in einer Menge von 67 % der theoretischen Ausbeute erhalten.

### Beispiel 3

Ein Gemisch aus 150 g 4-Acetamidophenol und 150 ml Diethylenglykoldimethylether wurde in Gegenwart von 3 g Pd (5 Gew.-%) auf Aktivkohle und unter Zusatz von 0,5 g Borax bei 160°C und einem Druck von 10 bar hydriert. Nach Aufnahme von 90 l Wasserstoff wurde die Hydrierung unterbrochen. Der gaschromatographischen Analyse zufolge war 4-Acetamidocyclohexanon in einer Selektivität von 80 % entstanden. Das Produkt kristallisierte beim Abkühlen des Reaktionsansatzes aus und konnte in einer Menge von 70 % der theoretischen Ausbeute erhalten werden.

## Patentansprüche

1. Verfahren zur Herstellung von substituierten Cyclohexanonen der Formel 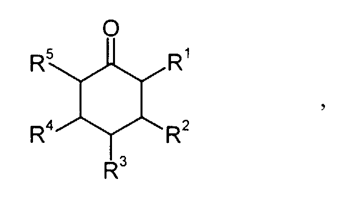 in der
R¹, R², R⁴ und R⁵ für Wasserstoff und R³ für Hydroxy, C₁-C₄-Alkoxy oder -NH-C₁-C₄-Acyl stehen,
durch heterogen katalysierte Hydrierung von substituierten Phenolen der Formel 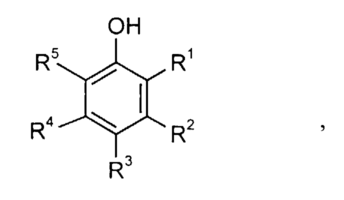 in der
R¹, R², R³, R⁴ und R⁵ die oben angegebene Bedeutung haben,
dadurch gekennzeichnet, daß die Hydrierung in Gegenwart eines gegebenenfalls auf einem Träger aufgebrachten Palladium-Katalysators sowie in Gegenwart eines oder mehrerer weiterer Zusatzstoffe aus der Reihe der alkalisch reagierenden (Erd)Alkali- oder Ammoniumsalze in einem Ether als Lösungsmittel durchgeführt wird.

2. Verfahren nach Anspruch 1, dadurch gekennzeichnet, daß die Hydrierung in Diethylenglykoldimethylether, Ethylenglykoldimethylether, 1,4-Dioxan, Tetrahydrofuran oder einem Gemisch mehrerer von ihnen durchgeführt wird.

3. Verfahren nach Anspruch 1, dadurch gekennzeichnet, daß Hydrochinon, 4-Methoxyphenol oder p-Acylaminophenol als Ausgangsmaterialien eingesetzt werden.

4. Verfahren nach Anspruch 1, dadurch gekennzeichnet, daß die Hydrierung in einem Temperaturbereich von 20°C bis 250°C, durchgeführt wird.

5. Verfahren nach Anspruch 1, dadurch gekennzeichnet, daß die Hydrierung in einem Druckbereich von 1 bar bis 200 bar durchgeführt wird.

6. Verfahren nach Anspruch 1, dadurch gekennzeichnet, daß als Kalatysator auf Aktivkohle aufgebrachtes Palladium verwendet wird.

7. Verfahren nach Anspruch 1, dadurch gekennzeichnet, daß als Zusatzstoffe Natriumcarbonat oder Borax verwendet werden.

## Claims

1. Process for preparing substituted cyclohexanones of the formula 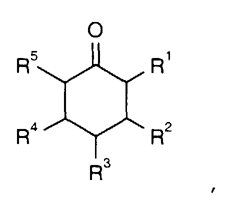 in which
R¹, R², R⁴ and R⁵ represent hydrogen and R³ represents hydroxy, C₁-C₄-alkoxy or -NH-C₁-C₄-acyl,
by heterogeneously catalysed hydrogenation of substituted phenols of the formula 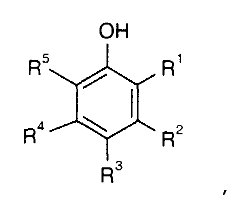 where
R¹, R², R³, R⁴ and R⁵ are as defined above,
characterized in that the hydrogenation is carried out in the presence of a palladium catalyst, optionally applied to a support, and also in the presence of one or more further additives selected from among the alkaline alkali metal, alkaline earth metal or ammonium salts in an ether as solvent.

2. Process according to Claim 1, characterized in that the hydrogenation is carried out in diethylene glycol dimethyl ether, ethylene glycol dimethyl ether, 1,4-dioxane, tetrahydrofuran or a mixture of a plurality thereof.

3. Process according to Claim 1, characterized in that hydroquinone, 4-methoxyphenol or p-acylaminophenol are used as starting materials.

4. Process according to Claim 1, characterized in that the hydrogenation is carried out in a temperature range from 20°C to 250°C.

5. Process according to Claim 1, characterized in that the hydrogenation is carried out in a pressure range from 1 bar to 200 bar.

6. Process according to Claim 1, characterized in that the catalyst used is palladium applied to activated carbon.

7. Process according to Claim 1, characterized in that additives used are sodium carbonate or borax.

## Revendications

1. Procédé pour la préparation de cyclohexanones substituées de formule 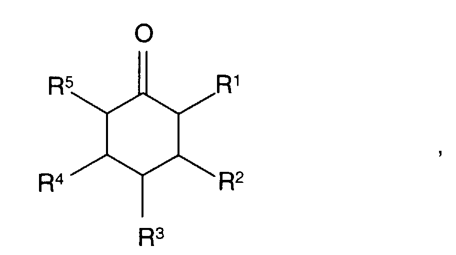 dans laquelle
R¹, R², R⁴ et R⁵ représentent l'hydrogène et R³ représente un groupe hydroxy, alkoxy en C₁-C₄ ou -NH-acyle en C₁-C₄, par hydrogénation avec catalyse hétérogène de phénols substitués de formule 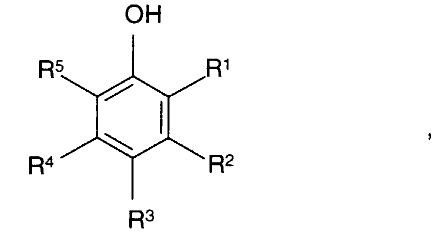 dans laquelle
R¹, R², R³, R⁴, et R⁵ ont les significations indiquées ci-dessus,
caractérisé en ce que l'hydrogénation est effectuée en présence d'un catalyseur au palladium éventuellement appliqué sur un support et en présence d'un ou plusieurs additifs de la classe des sels alcalins, alcalino-terreux ou d'ammonium à réaction alcaline dans un éther qui sert de solvant.

2. Procédé selon revendication 1, caractérisé en ce que l'hydrogénation est réalisée dans l'éther diméthylique du diéthylèneglycol, l'éther diméthylique de l'éthylèneglycol, le 1,4-dioxanne, le tétrahydrofuranne ou un mélange de plusieurs d'entre eux.

3. Procédé selon revendication 1, caractérisé en ce que le produit de départ mis en oeuvre est l'hydroquinone, le 4-méthoxyphénol ou un p-acylaminophénol

4. Procédé selon revendication 1, caractérisé en ce que l'hydrogénation est réalisée dans l'intervalle de température de 20°C à 250°C.

5. Procédé selon revendication 1, caractérisé en ce que l'hydrogénation est réalisée dans l'intervalle de pression de 1 à 200 bar.

6. Procédé selon revendication 1, caractérisé en ce que le catalyseur utilisé est le palladium appliqué sur charbon actif.

7. Procédé selon revendication 1, caractérisé en ce que l'additif utilisé est le carbonate de sodium ou le borax.
